# EUROPEAN PATENT APPLICATION

(11) **EP 4 151 080 A1**
(43) Date of publication of application: **22.03.2023**
(21) Application number: 21803556.6
(22) Date of filing: 11.05.2021
(51) Int. Cl.: A01G 18/20, A01G 18/30, A01G 18/66

(54) **CULTURE MEDIUM, FUNGUS BED, BAGGED FUNGUS BED, CULTURE MEDIUM MANUFACTURING METHOD, FUNGUS BED MANUFACTURING METHOD, BAGGED FUNGUS BED MANUFACTURING METHOD**

(30) Priority: 12.05.2020 JP 2020083951
(71) Applicant: Lala Corporation, Niigata-shi, Niigata 950-0943 (JP)
(72) Inventor: KOMABA, Hiromi, Niigata-shi, Niigata 950-0943 (JP)
(74) Representative: Marks & Clerk LLP
(86) International application number: PCT/JP2021/017931
(87) International publication number: WO 2021/230248

(57) **Abstract**

Provided are a culture medium, a fungal bed, and a bagged fungal bed each of which is less liable to have mold occurring therein, and methods of producing those. The culture medium of the present invention contains at least one or two or more of a *Bacillus* sp. bacterium, an *Ewingella* sp. bacterium, and a *Pseudomonas* sp. bacterium. The fungal bed of the present invention includes the culture medium inoculated with an inoculum, and the bagged fungal bed of the present invention includes the fungal bed accommodated in a film bag having fine perforations. The culture medium production method of the present invention is a method including the steps of: heating a substrate in a storage container; and supplying air into the storage container. The fungal bed production method of the present invention is a method including inoculating a culture medium, which is produced by the culture medium production method, with an inoculum of a mushroom, and the method of producing a bagged fungal bed of the present invention is a method including accommodating the fungal bed in a film bag having a plurality of fine perforations.

## Description

### Technical Field

The present invention relates to a culture medium to be used for fungal bed cultivation, a fungal bed including the culture medium inoculated with an inoculum of a mushroom, and a bagged fungal bed including the fungal bed accommodated in a bag, and to methods of producing those.

### Background Art

As a method of cultivating a mushroom, there is known fungal bed cultivation. In the fungal bed cultivation, a culture medium using a fungal bed including a wood substrate inoculated with an inoculum of a mushroom is mainly used. Hitherto, as the fungal bed to be used for the fungal bed cultivation, there has been known a bagged fungal bed including a fungal bed, which includes a culture medium inoculated with an inoculum of a mushroom, accommodated in a film bag having fine perforations (Patent Literature 1).

### Citation List

### Patent Literature

[PTL 1] JP 2016-041027 A

### Summary of Invention

### Technical Problem

The above-mentioned film bag has formed therein fine perforations, and when the fungal bed is poorly made, the fungal bed sometimes has a fungus (mold) other than a mushroom occurring therein owing to microorganisms invading through the fine perforations.

The present invention has been made in view of such circumstances, and an object to be achieved by the present invention is to provide a culture medium that is less liable to have mold occurring therein, a fungal bed including the culture medium inoculated with an inoculum of a mushroom, a bagged fungal bed including the fungal bed accommodated in a film bag, and methods of producing those.

### Solution to Problem

### [Culture Medium]

A culture medium according to the present invention contains at least one or two or more of a *Bacillus* sp. bacterium, an *Ewingella* sp. bacterium, and a *Pseudomonas* sp. bacterium. A method of producing the culture medium according to the present invention is not particularly limited, and for example, the culture medium may be produced by: a method involving sterilizing a culture medium prepared by a related-art method, and then adding one or two or more of a *Bacillus* sp. bacterium, an *Ewingella* sp. bacterium, and a *Pseudomonas* sp. bacterium; a method involving producing the culture medium through use of a raw material containing one or two or more of a *Bacillus* sp. bacterium, an *Ewingella* sp. bacterium, and a *Pseudomonas* sp. bacterium; or a culture medium production method according to the present invention to be described later.

### [Fungal Bed]

A fungal bed according to the present invention includes the above-mentioned culture medium inoculated with an inoculum of a mushroom.

### [Bagged Fungal Bed]

A bagged fungal bed according to the present invention includes the above-mentioned fungal bed accommodated in a film bag having a plurality of fine perforations.

### [Culture Medium Production method]

A culture medium production method according to the present invention is a method including the steps of: heating a substrate in a storage container; and supplying air into the storage container. In this method, for example, the substrate in the storage container is heated to kill fungi, microorganisms that are not thermophilic (hereinafter referred to as "non-thermophilic") (hereinafter referred to as "non-thermophilic microorganisms"), and microorganisms that are non-heat-resistant (hereinafter referred to as "non-heat-resistant microorganisms"), which are contained in the substrate in the storage container, and then air (preferably cool air) is supplied into the storage container to cool the substrate while microorganisms that are thermophilic (hereinafter referred to as "thermophilic microorganisms") and microorganisms that are heat-resistant (hereinafter referred to as "heat-resistant microorganisms") are allowed to remain, to thereby provide a culture medium. The culture medium production method according to the present invention may preferably produce a culture medium containing at least one or two or more of a *Bacillus* sp. bacterium, an *Ewingella* sp. bacterium, and a *Pseudomonas* sp. bacterium, but is not limited thereto.

### [Fungal Bed Production Method]

A fungal bed production method according to the present invention is a method of producing a fungal bed, including inoculating a culture medium, which is produced by the above-mentioned culture medium production method, with an inoculum of a mushroom.

### [Bagged Fungal Bed Production Method]

A method of producing a bagged fungal bed according to the present invention is a method of producing a bagged fungal bed, including accommodating a fungal bed, which is produced by the above-mentioned fungal bed production method, in a film bag having a plurality of fine perforations.

### Advantageous Effects of Invention

The culture medium of the present invention is excellent in resistance to external contamination and to an environment, and is less liable to have a fungus such as a mold occurring therein. The fungal bed and the bagged fungal bed each using the culture medium of the present invention also have similar effects. The fungal bed production method and the bagged fungal bed production method each using the culture medium production method of the present invention also have similar effects.

### Brief Description of Drawings

FIG. 1 is an explanatory view for illustrating an example of a bagged fungal bed of the present invention.
FIG. 2 is a flowchart for illustrating an example of a bagged fungal bed production method of the present invention.
FIG. 3 is an explanatory view for illustrating an example of an apparatus to be used for the bagged fungal bed production method and the like of the present invention.
FIG. 4 is an explanatory view of an indoor environment control system illustrated in FIG. 3 as viewed from a left side.
FIG. 5 is an explanatory view of the indoor environment control system illustrated in FIG. 4 as viewed from a planar side.
FIG. 6 includes contrasting photographs of a sterilized fungal bed and a fungal bed of the present invention.
FIG. 7 includes contrasting photographs of the sterilized fungal bed and the fungal bed of the present invention.
FIG. 8 includes photographs of the sterilized fungal bed on the 17th day of culture.
FIG. 9 includes photographs showing the results of a growth test.
FIG. 10 is a bacterial flora analysis flow.
FIG. 11 is a stacked bar graph for comparing bacterial flora distributions.
FIG. 12 is a bar graph showing the distribution of *Pseudomonas fluorescens.*
FIG. 13 is a bar graph showing the distribution of *Bacillus ginsengihumi.*
FIG. 14 is a bar graph showing the distribution of *Ewingella americana.*

### Description of Embodiments

### (Embodiments of Culture Medium, Fungal Bed, and Bagged Fungal Bed)

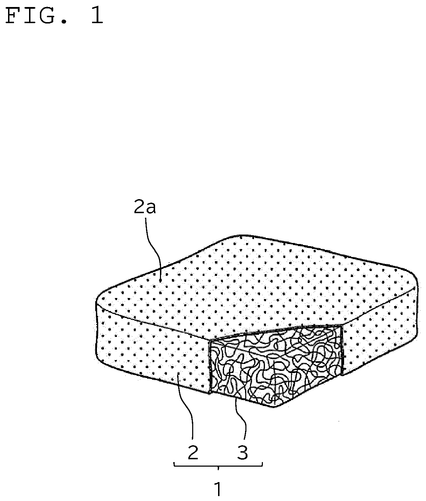

Examples of a culture medium, a fungal bed, and a bagged fungal bed according to embodiments of the present invention are described with reference to the drawings. A bagged fungal bed 1 illustrated in FIG. 1 as an example includes a film bag 2 and a fungal bed 3 accommodated therein. The film bag 2 having the fungal bed 3 accommodated therein is sealed by thermal welding or the like.

The film bag 2 of this embodiment has a plurality of fine perforations 2a. The fine perforations 2a are perforations for supplying air to the fungal bed. The size of each of the fine perforations 2a formed in the film bag 2 is preferably set to a diameter of from about 0.1 mm to about 1 um. However, the fine perforations 2a may be larger or smaller than the foregoing.

In this embodiment, a case in which, as the film bag 2, a black film bag 2 made of polyethylene is used is taken as an example. However, the film bag 2 may be made of a material other than polyethylene, such as a biodegradable material or polypropylene. A bag suited for the kind and characteristics of a mushroom is preferably used for the film bag 2. For example, when the mushroom is of a kind that requires exposure to light during culture, a transparent bag is preferably used, and when the mushroom is of a kind whose culture is completed in a dark place alone, a bag of a color having a light-blocking property (property of blocking light) or a color having a light-absorbing property (property of absorbing light), such as brown or dark blue, is preferably used.

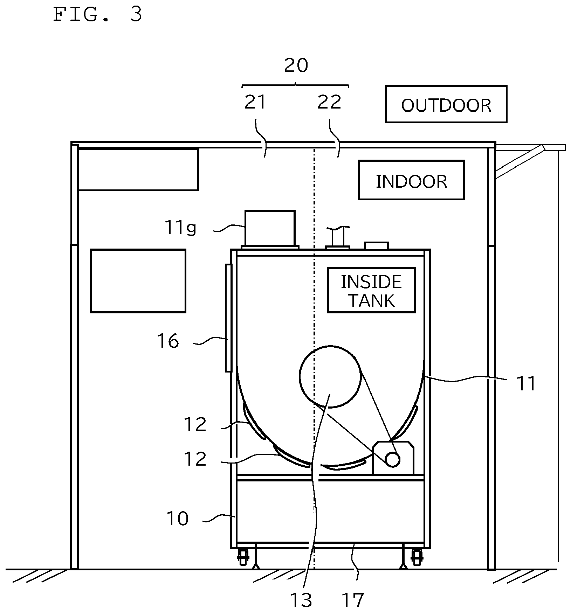

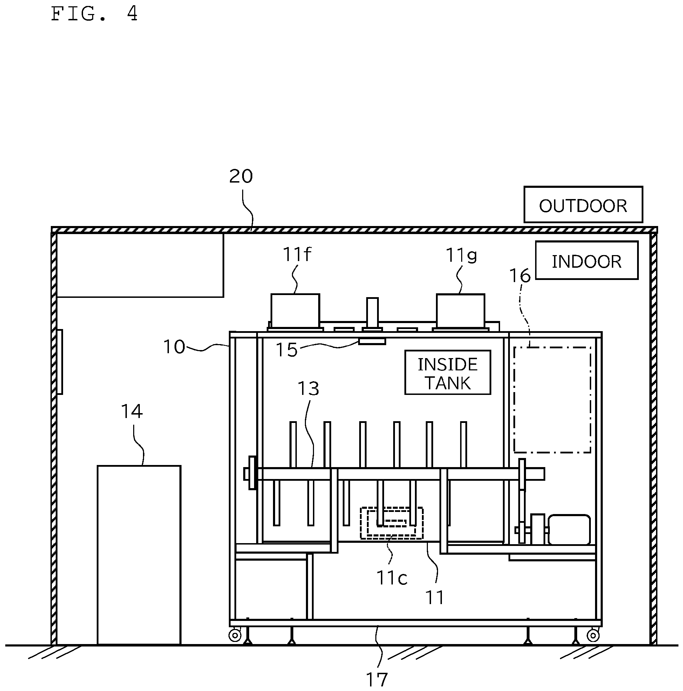

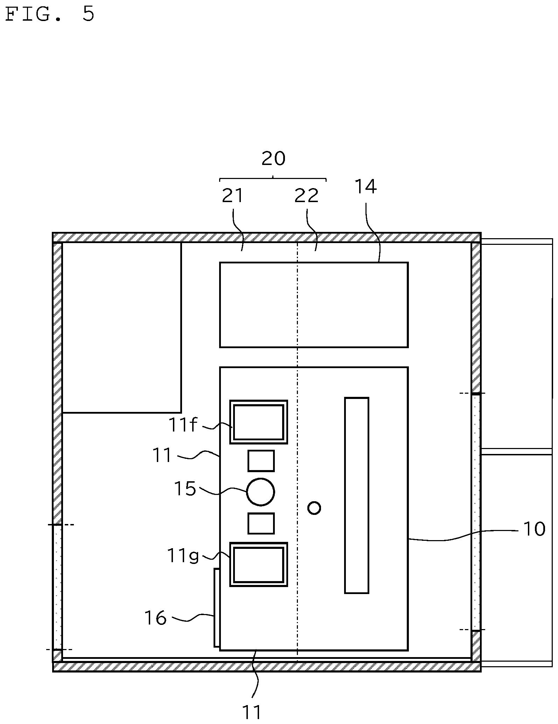

The fungal bed 3 is a fermented fungal bed including an anaerobically fermented and aerobically fermented culture medium inoculated with an inoculum of a mushroom. The fungal bed 3 according to this embodiment is a product obtained by: stirring a substrate having added thereto water in a storage container 11 (FIG. 3 to FIG. 5) having its temperature controlled; and inoculating the thus anaerobically fermented and aerobically fermented culture medium with an inoculum of a mushroom.

For example, a non-wood agricultural by-product is preferably used for the substrate. Examples of the "non-wood agricultural by-product" as referred to herein include cotton hulls, beet pulp, corncob, rice straw, peanut hulls, rice hulls, and wheat chaff. However, the substrate may be other than the foregoing.

In this embodiment, the culture medium is inoculated with, as the inoculum, an inoculum of *Pleurotus ostreatus.* The inoculum may be an inoculum of a mushroom other than *Pleurotus ostreatus,* such as shiitake, maitake, shimeji, nameko, enokitake, or *Pleurotus eryngii,* but is suitably an inoculum of a mushroom (wood-decay fungus) other than a mycorrhizal fungus. However, in this technology, a conceivable mechanism by which the effect of the present invention is exhibited is that a microflora existing in nature (mainly soil) and components produced through fermentation by the microflora contribute to the growth of the mushroom fungus, and hence the inoculum may be of a mushroom of a mycorrhizal fungus, such as matsutake, *Suillus bovinus, Rhizopogon roseolus,* a truffle, or *Lyophyllum shimeji.*

The inoculation with the inoculum is performed in the same storage container 11 as the storage container 11 used for the production of the culture medium. Specifically, the culture medium in the storage container 11 is cooled, and then inoculated with a predetermined amount of the inoculum. The culture medium inoculated with the inoculum is stirred and mixed in the storage container 11.

The finished culture medium and fungal bed each preferably contain at least one or two or more of a *Bacillus* sp. bacterium, an *Ewingella* sp. bacterium, and a *Pseudomonas* sp. bacterium, which are not contained in a related-art culture medium or fungal bed.

Examples of the *Bacillus* sp. bacterium include *Bacillus ginsengihumi, Bacillus* sp. YX, and *Bacillus cereus,* an example of the *Ewingella* sp. bacterium is *Ewingella americana,* and an example of the *Pseudomonas* sp. bacterium is *Pseudomonas fluorescens.*

The *Bacillus* sp. bacterium, the *Ewingella* sp. bacterium, the *Pseudomonas* sp. bacterium, or the like in the present invention may be added, or a bacterium adhering to a raw material may be utilized.

The culture medium, the fungal bed, and the bagged fungal bed of the present invention are each improved in resistance to external contamination and to an environment, and the fungal bed is less liable to have a fungus such as a mold occurring therein both during culture and after the completion of culture. A conceivable mechanism by which such effect is obtained is, for example, as follows: the action of the *Bacillus* sp. bacterium, the *Ewingella* sp. bacterium, or the *Pseudomonas* sp. bacterium causes the microflora in the culture medium and particularly the mushroom fungus and the like to preferentially proliferate.

(Embodiments of Culture Medium Production Method, Fungal Bed Production Method, and Bagged Fungal Bed Production Method)

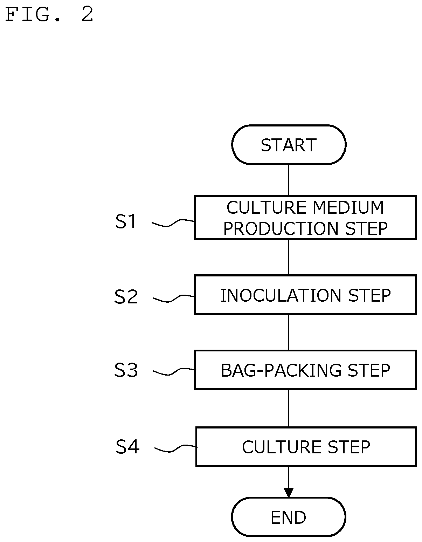

Next, examples of a culture medium production method, a fungal bed production method, and a bagged fungal bed production method according to embodiments of the present invention are described with reference to the drawings. Here, a method of producing the bagged fungal bed illustrated in FIG. 1 is taken as an example. In the bagged fungal bed production method according to this embodiment, as illustrated in FIG. 2, the bagged fungal bed is produced through a culture medium production step S1, an inoculation step S2, a bag-packing step S3, and a culture step S4.

The bagged fungal bed production method according to this embodiment may be carried out, for example, using such a fermented fungal bed production mixer (fungal bed production device) 10 illustrated in FIG. 3 to FIG. 5. In FIG. 3 to FIG. 5, reference numeral 11 denotes a storage container, 12 denotes heating means, 13 denotes stirring means, 14 denotes air blowing means, 15 denotes a temperature sensor, 16 denotes a controller, and 17 denotes a rack. The fungal bed production device 10 is installed in a small room 20 partitioned into a clean area 21 and an unclean area 22.

The culture medium production step S1 is a step of producing a culture medium serving as the base of a fungal bed. In this step, a substrate and water (including hot water. The same applies hereinafter.) are loaded into the storage container 11, and the substrate and the like are stirred by the stirring means 13 to produce a culture medium. Through this step, an anaerobically fermented or aerobically fermented culture medium is finished.

The above-mentioned cotton hulls, beet pulp, corncob, rice straw, peanut hulls, wheat chaff, or the like may be used for the substrate. The culture medium contains one or two or more of a *Bacillus* sp. bacterium, an *Ewingella* sp. bacterium, and a *Pseudomonas* sp. bacterium, which are not contained in a related-art culture medium.

This step is performed while the substrate in the storage container 11 is heated, for example, while the substrate is heated to such a temperature that fungi, non-thermophilic microorganisms, and non-heat-resistant microorganisms contained in the substrate are killed. The heating of the substrate may be performed by any of various methods, but in this embodiment, a case in which a substrate having added thereto water in the storage container 11 is heated by the heating means 12 (planar heater) is taken as an example. When heating is performed by this method, water contained in the substrate in the storage container 11 is vaporized to generate water vapor in the storage container 11. The water vapor heats the substrate in the storage container 11, and the heat kills fungi, non-thermophilic microorganisms, and non-heat-resistant microorganisms.

Temperatures at which fungi, non-thermophilic microorganisms, and non-heat-resistant microorganisms are killed are said to be generally from about 75°C to about 85°C, though varying depending on their kinds. Accordingly, in this step, it is appropriate to perform the heating to from 75°C to 85°C, at which fungi and non-thermophilic microorganisms are killed, preferably about 90°C, more preferably about 100°C.

The heated culture medium is cooled after the passage of a period (keeping time) for which its temperature is maintained. Specifically, air, preferably cool air, specifically cool air at 30°C or less (e.g., 20°C or less or 10°C or less) may be supplied into the storage container 11 from the air blowing means 14 to cool the culture medium with the cool air to a predetermined temperature. The cool air is introduced through a cool air inlet 11f arranged on the storage container 11, and is discharged through a cool air outlet 11g. The culture medium is preferably cooled to a temperature appropriate for the culture of the fungus body of a mushroom. The cooling is performed while the culture medium in the storage container 11 is stirred by the stirring means 13.

The inoculation step S2 is a step of inoculating the culture medium, which has been produced in the culture medium production step S1, with an inoculum. In this step, the inoculum is loaded into the storage container 11, and the culture medium loaded with the inoculum is stirred again. For the inoculum, there may be used an inoculum of *Pleurotus ostreatus* as well as a mushroom, such as shiitake, maitake, *Hypsizygus marmoreus,* nameko, enokitake, *Pleurotus eryngii,* matsutake, *Suillus bovinus, Rhizopogon roseolus,* a truffle, or *Lyophyllum shimeji.* Through this step, a fermented fungal bed is finished.

The fungal bed preferably contains one or two or more of a *Bacillus* sp. bacterium, an *Ewingella* sp. bacterium, and a *Pseudomonas* sp. bacterium, which are not contained in a related-art fungal bed. The *Bacillus* sp. bacterium, the *Ewingella* sp. bacterium, and the *Pseudomonas* sp. bacterium encompass those described in the embodiments of the culture medium, the fungal bed, and the bagged fungal bed. In addition to the foregoing, the fungal bed contains a microflora including, for example, various heat-resistant microorganisms and thermophilic microorganisms.

The bag-packing step S3 is a step of packing the fermented fungal bed, which has been finished through the inoculation step S2, into the film bag 2. In this step, the fermented fungal bed, which has been finished through the inoculation step S2, is taken out of a fungal bed outlet 11c of the storage container 11, and is accommodated in the film bag 2. The film bag having the fermented fungal bed accommodated therein is sealed by thermal welding or the like. The above-mentioned bag having the fine perforations 2a, specifically, for example, a colored or colorless and transparent bag suited for the kind and characteristics of the mushroom may be used for the film bag 2. The bag-packing may be performed using an apparatus (not shown), or may be performed manually.

The culture step S4 is a step of culturing the fungus body in the bagged fungal bed 1, which has been finished through the bag-packing step S3. In this step, the finished bagged fungal bed 1 is transferred to a culture chamber (not shown) having, for example, its temperature and humidity controlled, and hyphae in the bagged fungal bed 1 are grown and matured in the culture chamber.

The bagged fungal bed production method described herein is merely an example, and the culture medium production method, the fungal bed production method, and the bagged fungal bed production method of the present invention may also be carried out by other methods.

### <Example 1>

A comparative test was performed between a fungal bed produced by a related-art fungal bed production method (hereinafter referred to as "sterilized fungal bed") and a fungal bed produced by the fungal bed production method of the present invention (hereinafter referred to as "fungal bed of the present invention"). The overview and results of the comparative test are as described below.

### [Overview of Comparative Test]

### (1) Production of Sterilized Fungal Bed

1a. A substrate and hot water loaded into a bucket were kneaded to produce a culture medium. The substrate and hot water used are the same as the substrate and hot water of the fungal bed of the present invention of (2) to be described later.

1b. The culture medium produced in 1a above was placed in a bottle for an inoculum and was sterilized in an autoclave for 6 hours, and then the sterilized culture medium was cooled in a clean booth while being hermetically sealed.

1c. The culture medium cooled in 1b above was transferred to the film bag 2 having the fine perforations 2a and was then inoculated with an inoculum of *Pleurotus ostreatus* in the film bag 2, and the contents were mixed to produce a sterilized fungal bed. The film bag 2 having the sterilized fungal bed accommodated therein was sealed by thermal welding. The operation described here was also performed in the above-mentioned clean booth.

### (2) Production of Fungal Bed of the Present Invention

2a. A culture medium was produced by the culture medium production method of the present invention. Specifically, a substrate and hot water were loaded into the storage container 11, and the substrate and the like were stirred by the stirring means 13 to produce the culture medium. For the substrate and the hot water, the same ones as in the sterilized fungal bed of (1) in the foregoing were used.

2b. The substrate and the hot water in the storage container 11 were heated by the heating means 12, and were stirred at a certain speed by the stirring means 13 so that a temperature was applied to unformed organic matter as uniformly as possible. After the stirring, the temperature of the substrate was maintained for a certain period of time, followed by cooling in the same storage container 11 as that at the time of the production of the culture medium. Specifically, cool air was supplied into the storage container 11 from the air blowing means 14, and the culture medium was cooled with the cool air.

2c. The cooled culture medium in the storage container 11 was inoculated with an inoculum of *Pleurotus ostreatus,* and then the culture medium after the inoculation was stirred by the stirring means 13 to produce the fungal bed of the present invention.

2d. The produced fungal bed of the present invention was accommodated in the film bag 2 having the fine perforations 2a, and the film bag 2 after the accommodation was sealed by thermal welding. The operation described here was performed in the above-mentioned clean booth.

### (3) Culture

The sterilized fungal bed and the fungal bed of the present invention prepared in (1) and (2) in the foregoing were cultured under identical conditions.

### [Results of Comparative Test]

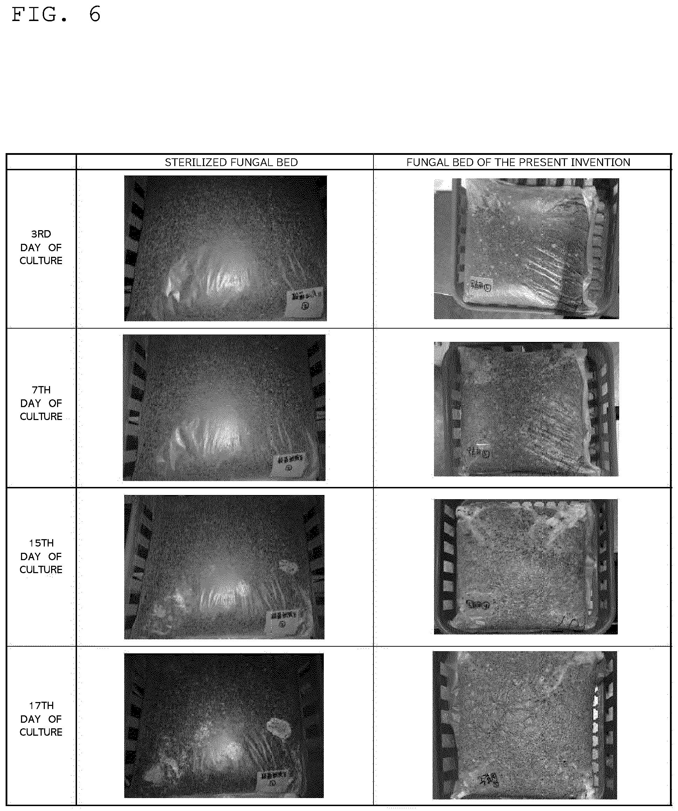

The results of this comparative test are shown in FIG. 6 to FIG. 8.

FIG. 6 includes contrasting photographs of the sterilized fungal bed and the fungal bed of the present invention, in which the uppermost row shows photographs on the 3rd day of culture, the second row shows photographs on the 7th day of culture, the third row shows photographs on the 15th day of culture, and the lowermost row shows photographs on the 17th day of culture.

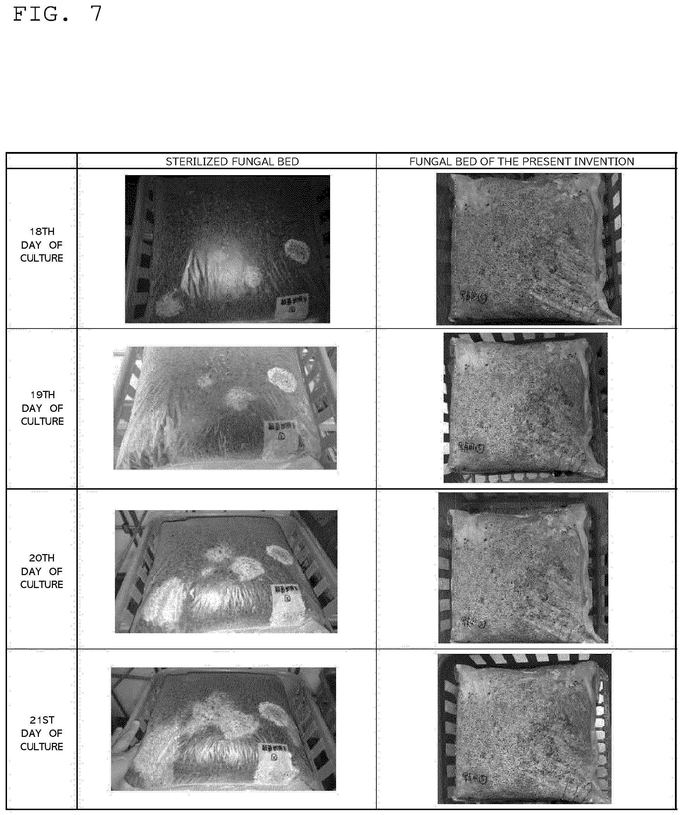

FIG. 7 includes contrasting photographs of the sterilized fungal bed and the fungal bed of the present invention, in which the uppermost row shows photographs on the 18th day of culture, the second row shows photographs on the 19th day of culture, the third row shows photographs on the 20th day of culture, and the lowermost row shows photographs on the 21st day of culture.

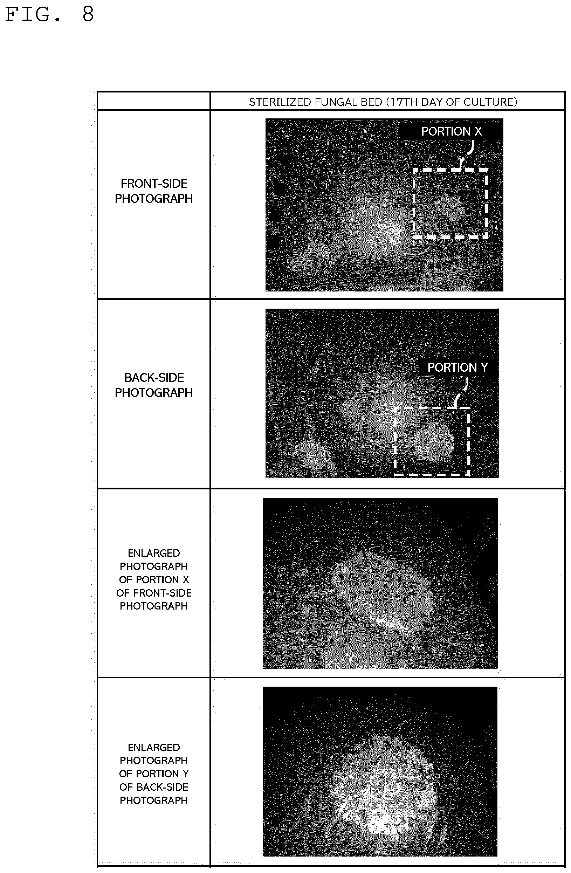

FIG. 8 includes photographs of the sterilized fungal bed on the 17th day of culture where the occurrence of mold was recognized, in which the uppermost row shows a front-side photograph thereof, the second row shows a back-side photograph thereof, the third row shows an enlarged photograph of the portion X of the front-side photograph shown in the uppermost row, and the lowermost row shows an enlarged photograph of the portion Y of the front-side photograph shown in the second row.

As shown in FIG. 6 to FIG. 8, in the sterilized fungal bed, although rhizomorphs of the *Pleurotus ostreatus* fungus were found in parts around the 15th day of culture, green-colored mold like *Trichoderma* was also found covering the top of the *Pleurotus ostreatus* fungus, and the proliferation of the mold was also recognized thereafter. Meanwhile, in the fungal bed of the present invention, rhizomorphs of the *Pleurotus ostreatus* fungus began to slightly appear from around the 3rd day of culture, the rhizomorphs spread along with the passage of days, and it was recognized on the 21st day of culture that the rhizomorphs were spread across the entire fungal bed.

### <Example 2>

A mushroom growth test was performed using the fungal bed (bagged fungal bed) of the present invention. The overview and results of the growth test are as described below. This test is a test that was performed separately from Example 1 described above.

### [Overview of Growth Test]

### (1) Preparation of Bagged Fungal Bed

1a. A culture medium was produced by the culture medium production method of the present invention. Specifically, a substrate and hot water were loaded into the storage container 11, and the substrate and the like were stirred by the stirring means 13 to produce the culture medium.

1b. The temperature of the culture medium during the production was maintained for a certain period of time, followed by cooling in the same storage container 11 as that at the time of the production of the culture medium. Specifically, cool air was supplied into the storage container 11 from the air blowing means 14, and the culture medium was cooled with the cool air.

1c. The cooled culture medium in the storage container 11 was inoculated with an inoculum of *Pleurotus ostreatus,* and the culture medium after the inoculation was stirred by the stirring means 13 to produce a fungal bed.

1d. The produced fungal bed was accommodated in the film bag 2 having the fine perforations 2a, and the film bag 2 after the accommodation was sealed by thermal welding. This operation was performed in a clean booth.

### (2) Culture

The bagged fungal bed prepared in (1) in the foregoing was cultured for a predetermined period, and then *Pleurotus ostreatus* was grown. In order not to impair the growth of *Pleurotus ostreatus,* incisions were made in the bagged fungal bed on the 22nd day after culture.

### [Results of Growth Test]

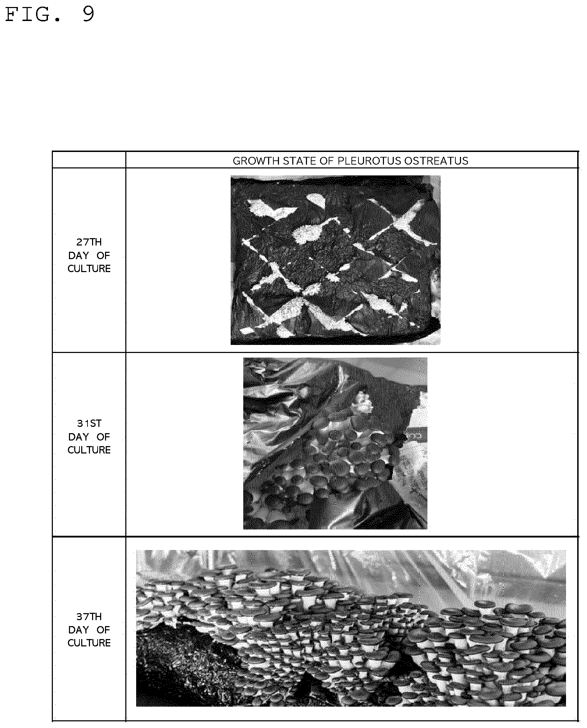

The results of this growth test are shown in FIG. 9. FIG. 9 includes photographs showing the state of growth of *Pleurotus ostreatus* using the bagged fungal bed prepared in (1) in the foregoing, in which the uppermost row shows a photograph on the 27th day of culture, the second row shows a photograph on the 31st day of culture, and the third row shows a photograph on the 37th day of culture.

As shown in FIG. 9, the growth of sprouts of *Pleurotus ostreatus* (serving as origins for fruiting bodies) was able to be recognized from the incisions formed in the film bag 2 on the 27th day of culture, the growth of *Pleurotus ostreatus* (fruiting bodies) was able to be recognized on the 31st day of culture, and *Pleurotus ostreatus* that had grown to such a degree as to be harvestable was recognized on the 37th day of culture.

Through this growth test, it was able to be recognized that, in the culture medium produced by the culture medium production method of the present invention and the bagged fungal bed produced by the fungal bed production method of the present invention, *Pleurotus ostreatus* (fruiting body) grew without the occurrence of mold inhibiting the growth of *Pleurotus ostreatus* even in, for example, an agricultural house containing many airborne bacteria as well.

### <Example 3>

Genetic analysis was performed for the culture medium produced by the culture medium production method of the present invention and the fungal bed produced by the fungal bed production method of the present invention. The overview and results of the analysis are described below.

### [Overview of Analysis]

### (1) Preparation of Samples

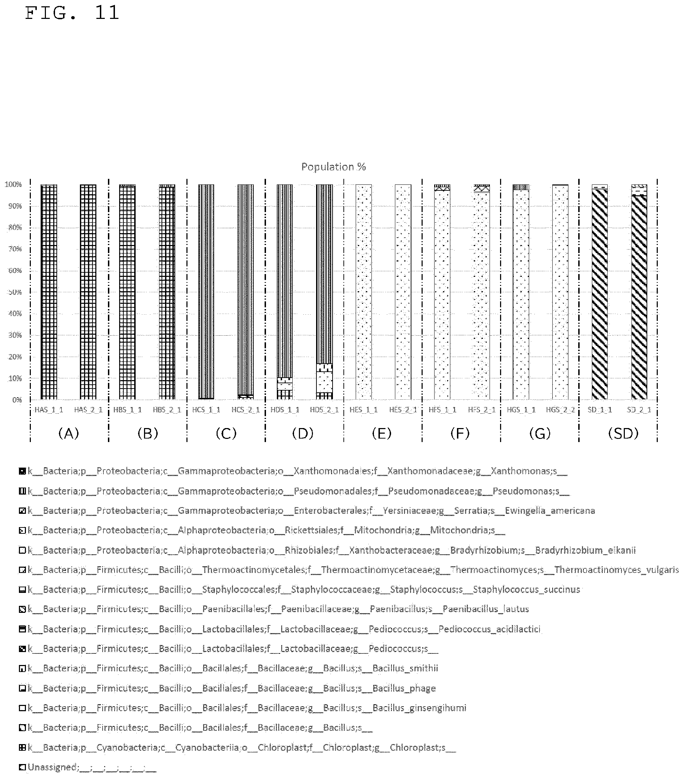

As samples, two each of the following samples (14 samples in total) were prepared: (A) ones after the loading of a substrate and the like and before heating; (B) ones after heating and before proceeding to a keeping time; (C) ones after the passage of the keeping time and before proceeding to cooling; (D) ones at the time of the completion of fermentation; (E) ones on the 3rd day of culture; (F) ones on the 7th day of culture; and (G) ones on the 15th day of culture. "SD_1-1" and "SD_2-1" in FIG. 11 to FIG. 14 represent cases in which the samples (D) were cultured in Petri dishes.

### (2) Acquisition of Amplicon Sequencing Data

For each sample, amplicon sequencing data on 16S ribosomal DNA was acquired.

### (3) Sequencing and Bacterial Flora Analysis

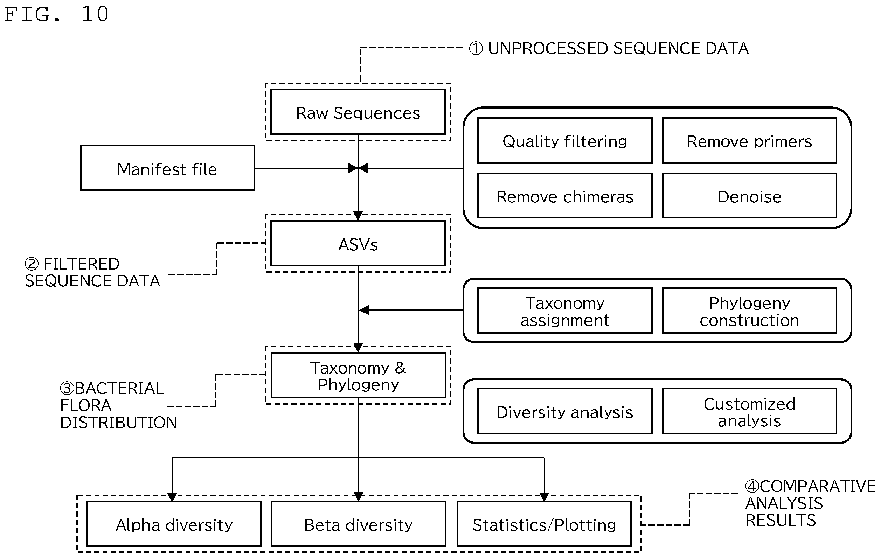

The DNA sequence of 16S ribosomal RNA obtained from each sample was sequenced with a sequencer PacBio, and then bacterial flora analysis was performed on the basis of a flow illustrated in FIG. 10.

### [Results of Analysis]

FIG. 11 shows a stacked bar graph for comparing bacterial flora distributions obtained as a result of the analysis. The stacked bar graph represents the distribution of a bacterial flora in each sample in percentage. Through this analysis, it was recognized that the fungal bed of the present invention contained 14 kinds of bacteria shown in Table 1. Table 1 is a list of bacteria each of which was contained in any one of the samples, and does not show that all the samples each contained all the 14 kinds of bacteria.

**Table 1**

| | |
|---|---|
| 1 | k_Bacteria;p_Cyanobacteria;c_Cyanobacteriia;o_Chloroplast;f_Chloroplast;g_Chloroplast;s_ |
| 2 | k_Bacteria;p_Firmicutes;c_Bacili;o_Baciliales;f_Bacillaceae;g_Bacillus;s_ |
| 3 | k_Bacteria;p_Firmicutes;c_Bacilli;o_eacillales;f_Bacillaceae;g_Bacillus;s_Bacillus_ginsengihumi |
| 4 | k_Bacteria;p_Firmicutes;c_Bacilli;o_Bacillales;f_Bacillaceae;g_Bacillus;s_Bacillus_phage |
| 5 | k_Bacteria;p_Firmicutes;c_Bacilli;o_Baciliales;f_Bacillaceae;g_Bacillus;s_Bacillus_smithii |
| 6 | k_Bacteria;p_Firmicutes;c_Bacilli;o_Lactobacillales;f_Lactobacillaceae;g_Pediococcus;s_Pediococcus_acidilactici |
| 7 | k_Bacteria;p_Firmicutes;c_Bacilli;o_Paenibacillales;f_Paenibaciilaceae;g_Paenibacillus;s_Paenibacillus_lautus |
| 8 | k_Bacteria;p_Firmicutes;c_Bacilli;o_Staphylococcales;f_Staphylococcaceae;g_Staphylococcus;s_Staphylococcus_succinus |
| 9 | k_Bacteria;p_Firmicutes;c_Bacilli;o_Thermoactinomycetales;f_Thermoactinomycetaceae;a_Thermoactinomyces;s_Thermoactinomyces_vulgaris |
| 10 | k_Bacteria;p_Proteobacteria;c_Alphaproteobacteria;o_Rhizobiales;f_Xanthobacteraceae;g_Bradyrhizobium;s_Bradyrhizobium_elkanii |
| 11 | k_Bacteria;p_Proteobacteria;c-Alphaproteobacteria;o_Rickettsiales;f_Mitochondria;q_Mitochondria;s_ |
| 12 | k_Bacteria;p_Proteobacteria;c_Gammaproteobacteria;o_Enterobacterales;f_Yersiniaceae;g_Serratia;s_Ewingella_americana |
| 13 | k_Bacteria;p_Proteobacteria;c_Gammaproteobacteria;o_Pseudomonadales;f_Pseudomonadaceae;g_Pseudomonas;s_ |
| 14 | k_Bacteria;p_Proteobacteria;c_Gammaproteobacteria;o_Xanthomonadates;f_Xanthomonadaceae;g_Xanthamonas;s_ |

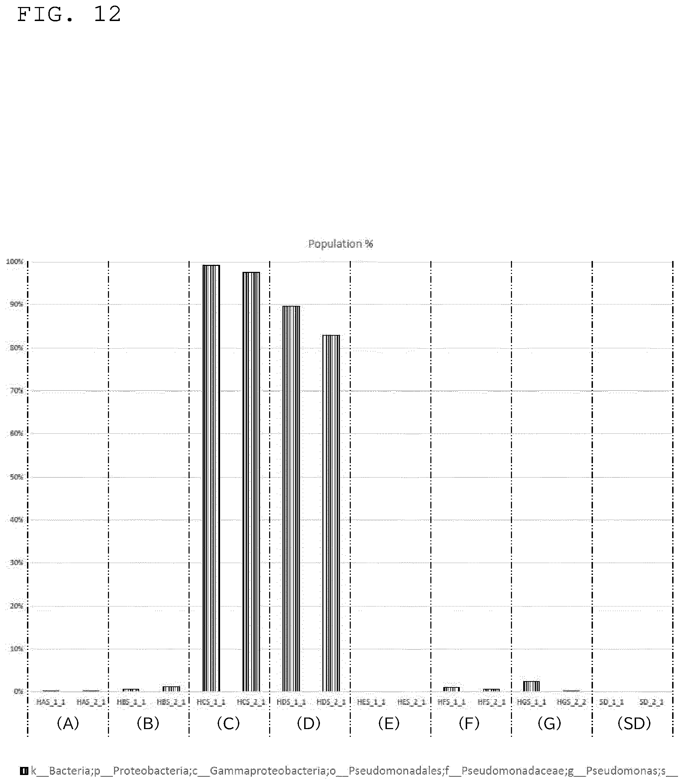

In the fungal bed produced by the fungal bed production method of the present invention, the ratio of *Pseudomonas* started to increase at the timing of (B) described above, and became a bacterial flora representing a vast majority at the timings of (C) and (D) described above before inoculation. After that, *Pseudomonas* was not recognized at the timing of (E) described above, but the presence thereof was recognized again at the timing of (F) described above. FIG. 12 shows a bar graph showing the distribution of *Pseudomonas fluorescens* in each sample.

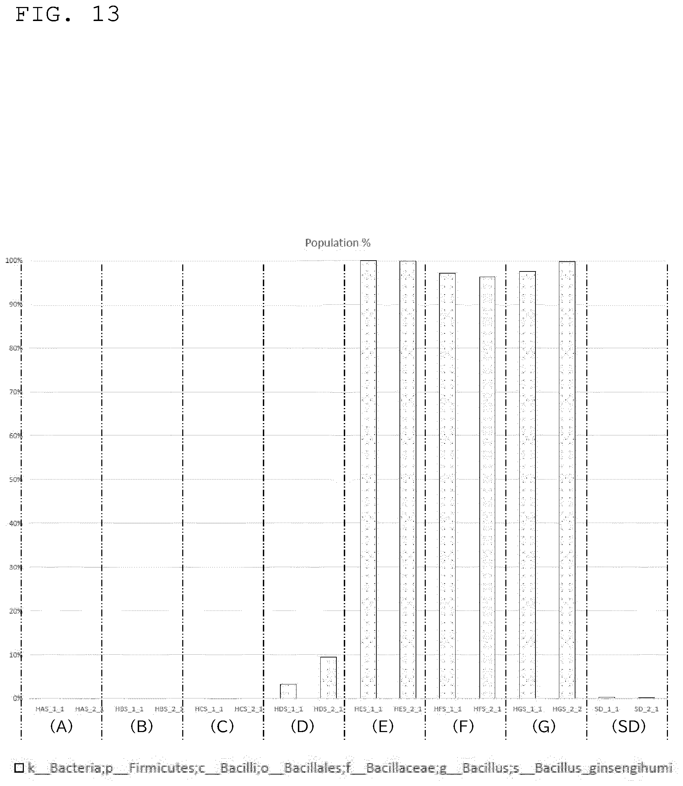

In the fungal bed produced by the fungal bed production method of the present invention, *Bacillus ginsengihumi* was recognized at the timing of (D) described above, and became a bacterial flora representing a vast majority at and after the timing of (E) described above after inoculation. FIG. 13 shows a bar graph showing the distribution of *Bacillus ginsengihumi* in each sample.

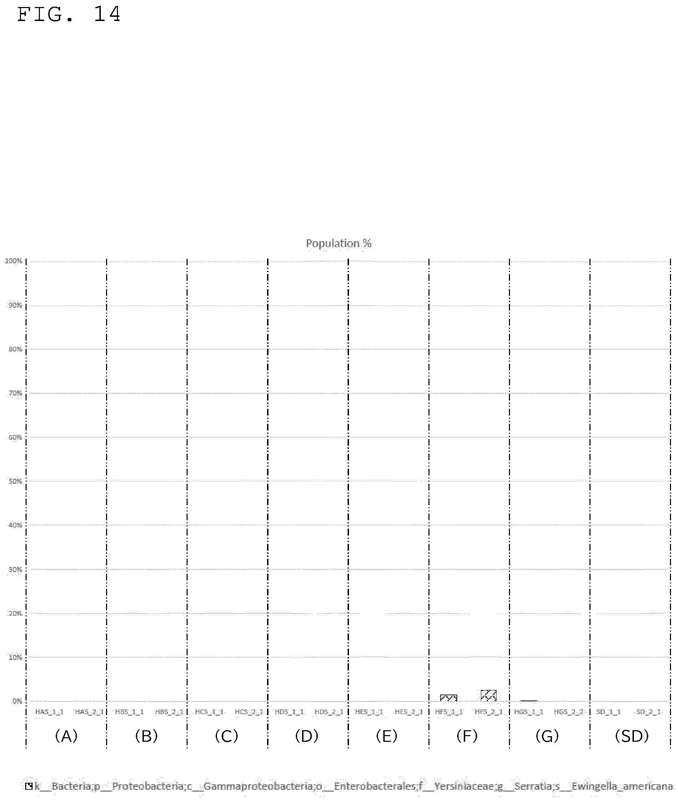

In the fungal bed produced by the fungal bed production method of the present invention, *Ewingella americana* was identified at the timing of (F) described above. *Ewingella americana* is a gram-negative nonsporulating facultative anaerobic rod, and is known as a shiitake rot disease pathogen. FIG. 14 shows a bar graph showing the distribution of *Ewingella americana* in each sample.

Through this analysis, a flora of various bacteria, which cause mold occurrence in general fungal bed cultivation, were detected in the culture medium produced by the culture medium production method of the present invention and the fungal bed produced by the fungal bed production method of the present invention. From the bacterial flora, *Pseudomonas* sp. bacteria, *Bacillus* sp. bacteria, and *Ewingella* sp. bacteria, which were considered to be harmful bacteria in general fungal bed cultivation, were identified.

In comprehensive consideration of the results of Examples described above, it is conceived that the culture medium, the fungal bed, and the bagged fungal bed of the invention of the present application contain various bacteria derived from the substrate, and the actions of those various bacteria allowed the mushroom to grow without the occurrence of mold in the fungal bed even under a situation in which airborne bacteria (e.g., *Trichoderma*) were able to adhere thereto.

### Reference Signs List

1 bagged fungal bed
2 film bag
2a fine perforation
3 fungal bed (fermented fungal bed)
10 fermented fungal bed production mixer (fungal bed production device)
11 storage container
11c fungal bed outlet
11f cool air inlet
11g cool air outlet
12 heating means
13 stirring means
14 air blowing means
15 temperature sensor
16 controller
17 rack
20 small room
21 clean area
22 unclean area

## Claims

1. A culture medium to be used for fungal bed cultivation,
the culture medium comprising at least one or two or more of a *Bacillus* sp. bacterium, an *Ewingella* sp. bacterium, and a *Pseudomonas* sp. bacterium.

2. A fungal bed to be used for fungal bed cultivation,
the fungal bed comprising the culture medium of claim 1 inoculated with an inoculum of a mushroom.

3. A bagged fungal bed to be used for fungal bed cultivation,
the bagged fungal bed comprising the fungal bed of claim 2 accommodated in a film bag having a plurality of fine perforations.

4. The bagged fungal bed according to claim 3,
wherein the film bag has a light-blocking property or/and a light-absorbing property.

5. A method of producing a culture medium to be used for fungal bed cultivation,
the method comprising the steps of:
heating a substrate in a storage container; and
supplying air into the storage container.

6. The method of producing a culture medium according to claim 5,
wherein the air supplied to the storage container is configured to cool the substrate in the storage container.

7. The method of producing a culture medium according to claim 5 or 6,
wherein the culture medium to be produced contains at least one or two or more of a *Bacillus* sp. bacterium, an *Ewingella* sp. bacterium, and a *Pseudomonas* sp. bacterium.

8. The method of producing a culture medium according to any one of claims 5 to 7,
wherein the step of heating a substrate in a storage container is performed so as to generate water vapor from water contained in the substrate.

9. A method of producing a fungal bed to be used for fungal bed cultivation,
the method comprising inoculating a culture medium, which is produced by the method of producing a culture medium of any one of claims 5 to 8, with an inoculum of a mushroom.

10. A method of producing a bagged fungal bed to be used for fungal bed cultivation,
the method comprising accommodating a fungal bed, which is produced by the method of producing a fungal bed of claim 9, in a film bag having a plurality of fine perforations.
